# EUROPEAN PATENT APPLICATION

(11) **EP 4 389 884 A1**
(43) Date of publication of application: **26.06.2024**
(21) Application number: 22216327.1
(22) Date of filing: 23.12.2022
(51) Int. Cl.: C12N 9/18, C12P 7/52

(54) **EFFICIENT PRODUCTION OF ENANTIOPURE D-3-HYDROXYBUTYRATE**

(71) Applicant: Schwab, Helmut, 8043 Graz (AT)
(72) Inventor: Schwab, Helmut, 8043 Graz (AT)
(74) Representative: Schwarz & Partner Patentanwälte GmbH

(57) **Abstract**

The present invention relates to a polyhydroxybutyrate depolymerase for producing enantiopure (R)-3-hydroxybutyrate acid or a salt thereof comprising an amino acid sequence having at least 80% identity to SEQ ID No. 2 and a motif comprising amino acid sequence ANLQXSKVYL (SEQ ID No. 5), wherein X is threonine, serine, glycine, glutamic acid, glutamine or aspartic acid.

## Description

### FIELD OF THE INVENTION

The present invention relates to polyhydroxybutyrate depolymerases for the production of enantiopure (R)-3-hydroxybutyrate.

### BACKGROUND ART

3-hydroxybutyrate is an important metabolite in living organisms. It is a so-called keto body and marketed as a food supplement in the form of different salts of 3-hydroxybutyric acid, for example as sodium, potassium, magnesium, calcium- or lithium salt. In addition, 3-hydroxybutyrate is also of interest for pharmaceutical applications.
3-hydroxybutyrate salts can be produced by chemical synthesis resulting in a racemic mixture of (*R*)- and (*S*)-hydroxybutyrate (alternatively also named as D- and L-hydroxybutyrate, respectively) salts. However, in living organisms including the human body, only the (*R*)-enantiomer is present. Therefore, it is of importance for food and pharmaceutical applications to provide the (*R*)- enantiomer, as the (*S*)- enantiomer does not have the intended function in biological systems and can have adverse effects on human and animal health if present in mixtures of (*R*)- and (*S*)-enantiomers.

In order to produce enantiopure (*R*)- hydroxybutyrate, fermentation processes employing engineered microbes can be established. Several methods are known in the art for the biotechnological production of 3-hydroxybutyrate. For instance, strategies to produce the enantiopure (*R*)-hydroxybutyrate are based on hydrolyzing the polymer beta-hydroxybutyrate (PHB) which is produced by many bacteria as a carbon storage compound. Biologically produced PHB consists solely of the (*R*)- hydroxybutyrate monomers linked to polymers via ester bonds between the carboxyl and the hydroxyl groups. PHB polymers can be produced by the fermentation of PHB producing bacteria.

Generally, the hydrolysis of PHB can be reached by two possible routes, chemical hydrolysis or enzyme catalyzed hydrolysis:
(i) Chemical hydrolysis involves a rather simple process but needs high concentrations of a strong base (e.g. NaOH, KOH) and high temperatures in order to obtain complete hydrolysis. This also means that it is needed to neutralize the reaction mixture by adding an acid which brings non-desired components into the reaction mixture. In case the hydrolysis is not coming to completion, a mixture of 3-hydroxybutyrate monomers and intermediate oligomers, which makes the purification process for gaining the pure 3-hydroxybutyrate monomers more complex. In addition, the 3-hydroxybutyrate monomer may be decomposed under such harsh conditions to crotonic acid, which represents an undesired compound as its odor is similar to butyric acid.
(ii) Enzymatic hydrolysis includes the cleavage of ester bonds of the polymer by successive cleaving off monomeric units. Enzymatic hydrolysis has been performed in vivo in combination with bacteria producing PHB and PHB-degrading enzymes. Such processes are characterized by the disadvantage that the produced 3-hydroxybutyrate ends up in a complex cultivation medium thus intense purification operations are needed.

However, reports so far show that the titers of enantiopure (*R*)-3-hydroxybutyrate salts in current procedures are quite low and thus, not economically feasible.

It is an object of the present invention to provide methods and means to efficiently produce enantiopure (*R*)-3-hydroxybutyrate.

### SUMMARY OF THE INVENTION

Thus, the present invention relates to a polyhydroxybutyrate depolymerase for producing enantiopure (*R*)-3-hydroxybutyrate acid or a salt thereof comprising an amino acid sequence having at least 80% identity to SEQ ID No. 2 and a motif comprising amino acid sequence ANLQXSKVYL (SEQ ID No. 5), wherein X is threonine, serine, glycine, glutamic acid, glutamine or aspartic acid.

It turned surprisingly out that the production of enantiopure (*R*)-3-hydroxybutyrate acid or a salt thereof can be significantly enhanced by the hydrolysis of polymers into (*R*)-3-hydroxybutyrate monomers by the polyhydroxybutyrate depolymerase of the present invention. In particular, the depolymerase of the present invention comprising the amino acid motif ANLQXSKVYL (SEQ ID No. 5), having threonine, serine, glycine, glutamic acid, glutamine or aspartic acid at position X is characterized by an enhanced enzymatic hydrolysis activity resulting in a superior product formation compared to other enzymes comprising different amino acids at this position of said motif. Hence, the motif appears to play an unexpected central role in the effective hydrolysis of PHB. Preferably, threonine at position X of the motif comprising amino acid sequence ANLQXSKVYL (SEQ ID No. 5) may have significant effect on the activity of the inventive enzyme. The polyhydroxybutyrate depolymerase according to the present invention can degrade polymers by cleaving chemical bonds between the monomers within the polymer chain and/or by cleaving chemical bonds crosslinking the chains of the polymer. The enzyme may act in a way that the monomeric (*R*)-3-hydroxybutyrate units are progressively cleaved off from one end of the polymer strands, resulting in an enantiopure (*R*)-3-hydroxybutyrate acid or a salt thereof.

Another aspect of the present invention relates to a vector encoding the polyhydroxybutyrate depolymerase as described above, wherein the nucleic acid molecule is operably linked to a promoter.

Another aspect of the present invention relates to a vector comprising the nucleic acid molecule.

Another further aspect of the present invention relates to a host cell comprising the nucleic acid molecule of and/or the vector as mentioned above.

Another aspect of the present invention relates to a method for producing enantiopure (*R*)-3-hydroxybutyrate acid or a salt thereof comprising the steps of:
a) providing at least one solid polyhydroxybutyrate polymer, and
b) incubating in an aqueous solution said at least one polymer
   - with at least one first polyhydroxybutyrate depolymerase and/or a host cell as mentioned above and/or
   - with at least one second polyhydroxybutyrate depolymerase comprising an amino acid sequence having at least 80% identity to SEQ ID No. 2 and a motif comprising an amino acid sequence ANLQNSKVYL (SEQ ID No. 10).

It turned out that the incubation of a solid polyhydroxybutyrate polymer with a first polyhydroxybutyrate depolymerase and/or a host cell and/or with a second polyhydroxybutyrate depolymerase as described above may result in an efficient process operation for producing enantiopure (R)-3-hydroxybutyrate acid or a salt thereof. The product formation can be maintained constant during the process since product inhibition of the polyhydroxybutyrate depolymerase of the present invention by (*R*)-3-hydroxybutyrate acid or a salt thereof can be circumvented. In particular, the first enzyme may be less inhibited at higher product concentrations, wherein the second enzyme may have a higher activity at low product concentrations. Therefore, the combination of the first and the second enzyme result in an efficient process operation during product formation since product inhibition can be avoided.

Another further aspect of the present invention relates to the use of a polyhydroxybutyrate depolymerase as mentioned above for producing enantiopure (*R*)-3-hydroxybutyrate acid or a salt thereof.

Yet another aspect of the present invention relates to a food supplement or a kit comprising a first polyhydroxybutyrate depolymerase and/or a second polyhydroxybutyrate depolymerase as defined above and a polyhydroxybutyrate polymer. 3-hydroxybutyrate is of interest as a keto nutrient. It turned surprisingly out that 3-hydroxybutyrate can be provided in the correct enantiomeric (R)- form when PHB is hydrolyzed by the first and/or second polyhydroxybutyrate depolymerase of the present invention in the form of a food supplement or a kit. The solid PHB polymer can be contacted with at least one polyhydroxybutyrate depolymerase of the present invention to induce hydrolysis of PHB into enantiopure (*R*)-3-hydroxybutyrate monomers. For instance, the solid PHB polymer and the at least one polyhydroxybutyrate depolymerase can be enveloped in capsules or coatings which are resistant to the stomach and get dissolved and released into the aqueous environment of the intestine. As the pH in the intestine is slightly alkaline and thus well suited for the action of the enzyme, the (*R*)-3-hydroxybutyrate monomers can get released, absorbed, and further metabolized. The benefit of this strategy in comparison to a direct application of the monomer as nutrient is, that the release of (*R*)-3-hydroxybutyrate from the polymer is in a slow manner and thus peaks of higher concentrations are avoided. Encapsulation can either be performed by standard acid-resistant capsules or in a micro-coating process.

### SHORT DESCRIPTION OF THE FIGURES

Fig.1 shows a SDS Polyacrylamide gel of supernatants of cultures of Acidovorax sp. BT293. Lane 1, size standard; Lanes 2-4, cultivation at 37°C; lanes 5-7, cultivation at 28°C; lanes 2 and 5, no addition; lanes 3 and 6, addition of 0.1 g/L 3-hydroxybutyrate; lanes 4 and 7, addition of 1 g/L PHB. The arrows indicate the protein band of polyhydroxybutyrate depolymerase.
Fig. 2 shows a comparison of the hydrolysis of PHB by different polyhydroxybutyrate depolymerases. The dotted lines represent 50% and 100% theoretical conversion of PHB into the 3-hydroxybutyrate monomers.
Fig. 3 shows the product formation rates at 70% conversion. TP0003 is not included as with this enzyme 70% conversion could not be reached over the observed time.
Fig. 4 shows 3-hydroxybutyrate monomer inhibition effects on TP0001 and TP0004.
Fig. 5 shows the effects of NaOH pre-treatment on hydrolysis activity of TP0001. The dotted lines represent 50% and 100% theoretical conversion of PHB into the 3-hydroxybutyrate monomers.
Fig. 6 shows the dependence of the hydrolysis rate of PHB-DP on pH in the reaction.

### EMBODIMENTS OF THE INVENTION

"% identity", as used herein, is obtained by aligning two amino acid sequences to be compared to give a maximum correlation between the sequences. This may include inserting "gaps" in either one or both sequences, to enhance the degree of alignment. A % identity may then be determined over the whole length of each of the sequences being compared. In the above context, an amino acid sequence having a "identity" of at least, for example, 80% to a query amino acid sequence, is intended to mean that the sequence of the subject amino acid sequence is identical to the query sequence except that the subject amino acid sequence may include up to twenty amino acid alterations per each 100 amino acids of the query amino acid sequence. In other words, to obtain an amino acid sequence having a sequence of at least 80% identity to a query amino acid sequence, up to 20% (20 of 100) of the amino acid residues in the subject sequence may be inserted or substituted with another amino acid or deleted.

Methods for comparing the identity of two or even more sequences are well known in the art. The percentage to which two sequences are identical can for example be determined by using a mathematical algorithm. Such algorithms are integrated in the BLAST family of programs, e.g. BLAST or NBLAST program, accessible through the home page of the NCBI
(www.ncbi.nlm.nih.gov).

The term "motif", as used herein, means a set of amino acids at specific positions along an aligned sequence of evolutionarily related proteins. Amino acids at specific positions are essential in the structure, the stability, or the activity of a protein.

Particularly preferred is a polyhydroxybutyrate depolymerase comprising an amino acid sequence having at least 80% identity to SEQ ID No. 2 and a motif comprising amino acid sequence ANLQXSKVYL (SEQ ID No. 5), wherein X is threonine. The polyhydroxybutyrate depolymerase of the present invention comprising the motif as mentioned above having threonine at position X was found to be profoundly effective in PHB degradation for the production of enantiopure (*R*)-3-hydroxybutyrate acid or a salt thereof. According to another preferred embodiment of the present invention, X is threonine.

According to another preferred embodiment of the present invention the motif may comprise amino acid sequence selected from the group consisting of VIDPVANLQXSKVYLFSGT (SEQ ID No. 6), GVIDPVANLQXSKVYLFSGTLDSVVKTGVM (SEQ ID No. 7), QGVIDPVANLQXSKVYLFSGTLDSVVKTGVMDALRTYYNSF (SEQ ID No. 8), and QGVIDPVANLQXSKVYLFSGTLDSVVKTGVMDALRTYYNSFVPAANVVYKKDIA (SEQ ID No. 9). X can be threonine, serine, glycine, glutamic acid, glutamine or aspartic acid.

According to another preferred embodiment of the present invention the amino acid sequence has at least 85% identity, preferably at least 90% identity, more preferably at least 95% identity, more preferably at least 99% identity, to SEQ ID No. 2.

Microbial strains producing polyhydroxybutyrate depolymerases are widespread in nature and different types of PHB depolymerizing enzymes have been described. Microbial polyhydroxybutyrate depolymerases can either be located intracellularly or can be secreted into the outside of the cells. Especially the cultivation of microbial strains which secrete polyhydroxybutyrate depolymerases provides a simple natural source for manufacturing enzyme preparations exhibiting depolymerase activity. It might be beneficial to induce biosynthesis of the polyhydroxybutyrate depolymerases in such natural strains as it makes sense to the organisms to only produce the enzyme when PHB or a related polymer is available as a nutrient source. Induction of polyhydroxybutyrate depolymerases biosynthesis can for example be performed by addition of PHB or 3-hydroxybutyrate salts to the cultivation medium.

According to another preferred embodiment of the present invention the polyhydroxybutyrate depolymerase is derived from a bacterium of the family *Comamonadaceae,* preferably of the genus Acidovorax, Delftia, Diaphorobacter, Comamonas, Rhodoferax, Caenimonas, Polaromonas, Ramlibacter, Giesbergeria, Xylophilus, Aquabacterium, Ideonella or Variovorax.

According to another preferred embodiment of the present invention the polyhydroxybutyrate depolymerase is derived from Acidovorax sp. BT293, Delftia acidovorans, Acidovorax sp. TP4, Acidovorax sp. KKS102, Acidovorax sp. BE320, Acidovorax sp. ST3, Acidovorax sp. HMWF01 or Acidovorax sp. Root568.

The current invention also provides a nucleic acid molecule encoding the polyhydroxybutyrate depolymerase as described above. The nucleic acid molecule can be operably linked to a promoter. As used herein, "operably linked" refers to a functional linkage between a promoter and the nucleic acid molecule encoding the depolymerase of the present invention, wherein the promoter sequence initiates and mediates transcription of the DNA sequence corresponding to said nucleic acid molecule.

The term "promoter", as used herein, refers to a region of a nucleic acid molecule upstream from the start of transcription and involved in recognition and binding of RNA polymerase and other proteins to initiate transcription. Promoters are able to control (initiate) transcription in a cell. Bacterial promoters are able of initiating transcription in bacterial cells whether or not its origin is a bacterial cell. The promoter used in the vector of the present invention can be "inducible" or "repressible", i.e. under environmental control. Such promoters can be controlled by changing the cultivation conditions (e.g. temperature) or by adding specific substances. The promoter used in the vectors of the present invention may be a "constitutive" promoter. Constitutive promoters are active under most environmental conditions and express continuously a protein or polypeptide of interest. In order to prevent transcriptional activation of downstream nucleic acid sequences by upstream promoters the vector of the present invention may comprise a "terminator" or "terminator sequence". The term "expression", as used herein, refers to the transcription and stable accumulation of sense (mRNA) or antisense RNA derived from the nucleic acid fragments of the invention. Expression may also refer to translation of mRNA into a polypeptide.

The nucleic acid molecule of the present invention can be part of a vector which can be used for cloning or expression purposes. Respective vectors are well-known in the art. The term "vector" refers to a DNA molecule used as a vehicle to transfer recombinant genetic material into a host cell. The major types of vectors are plasmids, bacteriophages, viruses, cosmids, and artificial chromosomes. Vectors called expression vectors (expression constructs) are specifically adapted for the expression of the heterologous sequences in the target cell, and generally have a promoter sequence that drives expression of the heterologous sequences encoding a polypeptide. Generally, the regulatory elements that are present in an expression vector include a transcriptional promoter, a ribosome binding site, a terminator, and optionally an operator. Preferably, an expression vector also contains an origin of replication for autonomous replication in a host cell, a selectable marker, a limited number of useful restriction enzyme sites, and a potential for high copy number. Examples of expression vectors are cloning vectors, modified cloning vectors, specifically designed plasmids and viruses. Expression vectors providing suitable levels of polypeptide expression in different hosts are well known in the art. Expression vectors may be introduced into host cells using standard techniques. Examples of such techniques include transformation, transfection, lipotransfection, protoplast fusion, and electroporation. The vector of the present invention can be designed to be integrated into the genome of the host cell. Alternatively, the vector is designed to not integrate into the genome of a host cell allowing transient expression of the nucleic acid molecule of the present invention. In the latter case the vector remains in a non-integrated state free within the cell.

According to the present invention a host cell comprises the nucleic acid molecule as described above. The host cell can be used to produce/express at least one protein or polypeptide of interest which is not naturally occurring in said host cell. In order to allow the recombinant expression of heterologous polypeptides and proteins the respective nucleic acid molecule encoding said polypeptides and proteins are introduced into the host cell of the present invention.

"Polymer", as used herein, refers to a substance which comprises two or more subunits ("monomers") which are covalently attached to each other to form a branched or unbranched chain. Two or more of such chains may be crosslinked, i.e. covalently bound to each other. The monomers forming the polymer may be identical or structurally different. Polymer comprising two or more of the same monomer are homopolymers, whereas polymers comprising two or more chemically distinct monomers are copolymers.

The method of the present invention can be performed in standard state of the art biochemical reaction processes. One option is using a stirred tank reactor in a batch mode. The solid polyhydroxybutyrate polymer can be suspended in an aqueous solution and at least one first polyhydroxybutyrate depolymerase and/or the at least one second polyhydroxybutyrate depolymerase can be added either as a solid preparation or as a liquid preparation. The reaction can be kept at a defined pH value by adding a base. Thus, the released enantiopure (*R*)- 3-hydroxybutyrate acid can be simultaneously neutralized by adding a proper base in stoichiometric amounts to the released acid in the aqueous solution. The base to be used depends on the desired final enantiopure (*R*)- 3-hydroxybutyrate salt product and can be for example NaOH, KOH, LiOH, Mg(OH)₂, Ca(OH)₂ in the form of concentrated aqueous solutions or as solids. A defined mixture of bases for production of a mixed enantiopure (*R*)- 3-hydroxybutyrate salt preparation can also be added to the mixture.

A second option would be the operation in a membrane reactor which allows the constant removal of the (*R*)- 3-hydroxybutyrate acid or salts thereof and retaining the polyhydroxybutyrate depolymerase in the reaction. In this process mode at least one solid polyhydroxybutyrate polymer and at least one first polyhydroxybutyrate depolymerase and/or a host cell and/or at least one second polyhydroxybutyrate depolymerase of the present invention are simultaneously added to the aqueous solution and the reaction is performed under controlled pH conditions by adding a suitable base as described above for the batch mode process. This provides the benefit of avoiding product inhibition to the polyhydroxybutyrate depolymerase by (*R*)- 3-hydroxybutyrate. In addition, this operation mode provides longer operation cycles. The use of membrane reactors is a standard technology in enzyme-catalyzed production processes. Suitable membrane types are commercially available and depending on the process needs; ultrafiltration membranes or small pore-sized microfilters from nanometer to micrometer pore size can be applied.

A further option is to combine chemical and enzymatic hydrolysis. In a first step a chemical hydrolysis step by a base at high pH and temperature can be used to pre-digest the least one solid polyhydroxybutyrate polymer and thereby reducing the chain-length of the polymer. In a second step the pH-controlled enzymatic hydrolysis by incubating the reduced polymer with at least one first polyhydroxybutyrate depolymerase and/or a host cell and/or with at least one second polyhydroxybutyrate depolymerase of the present invention would produce the enantiopure (*R*)- 3-hydroxybutyrate acid or a salt thereof.

According to another preferred embodiment of the present invention before and/or during step b) of the present invention the pH of the aqueous solution ranges from pH 5 to 10, preferably from pH 6 to 8, more preferably from pH 6.5 to 7.5.

A further advantage of the present invention is the use of weak bases (for example Ca(OH)₂ or Mg(OH)₂) as the base is needed only for neutralization for but not for ester bond cleavage. Thus, for example Ca- or Mg salts of (*R*)- 3-hydroxybutyrate can be directly produced by neutralizing with such bases which are of industrial interest. According to another preferred embodiment of the present invention the pH of the aqueous solution is adjusted by adding NaOH, KOH, LiOH, Mg(OH)₂, Ca(OH)₂ and/or NH₄OH to said aqueous solution.

Another important aspect of the present invention relates to the incubation of the at least one solid polyhydroxybutyrate polymer with one or more polyhydroxybutyrate depolymerases having different specific activities at different (*R*)- 3-hydroxybutyrate concentrations. For example, the at least one first polyhydroxybutyrate depolymerases as described above can have a high activity at high (*R*)- 3-hydroxybutyrate concentrations and can be combined with at least one second polyhydroxybutyrate depolymerases which can have a higher activity at lower (*R*)- 3-hydroxybutyrate concentrations but a decreased activity at higher product concentrations to reach better overall productivity along the hydrolysis process. The at least one second polyhydroxybutyrate depolymerases of the present invention is characterized having at least 80% identity to SEQ ID No. 2 and a motif comprising an amino acid sequence ANLQNSKVYL (SEQ ID No. 10).

According to another preferred embodiment of the present invention the motif of the at least one second polyhydroxybutyrate depolymerase comprises amino acid sequence selected from the group consisting of VIDPVANLQNSKVYLFSGT (SEQ ID No. 11), GVIDPVANLQNSKVYLFSGTLDSWKTGVM (SEQ ID No. 12), QGVIDPVANLQNSKVYLFSGTLDSVVKTGVMDALRTYYNSF (SEQ ID No. 13), and QGVIDPVANLQNSKVYLFSGTLDSVVKTGVMDALRTYYNSFVPAANVVYKKDIA (SEQ ID No. 14).

According to another preferred embodiment of the present invention the at least one first and/or the at least one second polyhydroxybutyrate depolymerase comprises a further motif comprising amino acid sequence GATANSMAIG (SEQ ID No. 15), or SGATANSMAIGW (SEQ ID No. 16). Polyhydroxybutyrate depolymerase comprising a further motif comprising SEQ ID No. 15 or SEQ ID No. 16 appeared to have a higher enzymatic activity at lower product concentrations of (*R*)- 3-hydroxybutyrate compared to enzymes lacking said further motif.

According to another preferred embodiment of the present invention the at least one second polyhydroxybutyrate depolymerase comprises or consists of an amino acid sequence having at least 85% identity, preferably at least 90% identity, more preferably at least 95% identity, more preferably at least 99% identity, to SEQ ID No. 2.

It also turned surprisingly out that the hydrolysis rate can be significantly increased by the pre-treatment of the PHB polymer before incubating with the polyhydroxybutyrate depolymerases and/or a host cell of the present invention.

According to another preferred embodiment of the present invention the at least one polyhydroxybutyrate polymer is pre-treated before step b) with an aqueous solution at a pH in the range of 8 to 14.

According to another preferred embodiment of the present invention the at least one polyhydroxybutyrate polymer is pre-treated before step b) with an aqueous solution having a temperature between 60°C and 140°C, preferably between 80°C and 100°C.

The preparations of the at least one first and the at least one second polyhydroxybutyrate depolymerase of the present invention can be obtained by cultivation using microbes by standard state of the art methods. In case the polyhydroxybutyrate depolymerase is produced intracellularly, cells can be harvested from the culture by centrifugation, filtration, precipitation or flotation processes, disrupted by an appropriate method (e.g. ultrasonic disintegrator, French press homogenizer, high pressure homogenizer) and the cell debris can be removed (e.g. by centrifugation). The cell lysate can further be separated into a soluble and an insoluble fraction prior to further processes. In case the polyhydroxybutyrate depolymerases is secreted into the culture medium, the cells can be removed from the culture by centrifugation, filtration, precipitation or flotation processes and the culture supernatant can be used for further processes. Further process steps for obtaining an appropriate enzyme preparation may include state of the art concentration steps including precipitation or ultrafiltration, chromatographic purification steps as ion exchange chromatography, hydrophobic interaction chromatography, affinity chromatography, size exclusion chromatography, buffer exchange steps and drying procedures as freeze drying, film evaporation, or spray drying. A further immobilization procedure can also be included if required.
The obtained polyhydroxybutyrate depolymerase of the present invention can either be incubated with at least one solid polyhydroxybutyrate polymer in an aqueous solution in the form of a crude preparation (e.g. as cell lysate, culture supernatant, concentrated and/or partially purified preparation thereof) or as a purified preparation, in liquid or solid form.

According to another preferred embodiment of the present invention the at least one first polyhydroxybutyrate depolymerase and/or the at least one second polyhydroxybutyrate depolymerase is provided in a solution or in a solid form. The solid polyhydroxybutyrate depolymerase can be provided in lyophilized or spray dried form. Methods for producing such enzyme preparations are well known in the art. The at least one first polyhydroxybutyrate depolymerase and/or the at least one second polyhydroxybutyrate depolymerase can also be suspended in an aqueous solution.

According to another preferred embodiment of the present invention the at least one first polyhydroxybutyrate depolymerase and/or the at least one second polyhydroxybutyrate depolymerase are provided in the form of a purified protein, a cell extract and/or as a culture supernatant.

According to another preferred embodiment of the present invention the at least one polyhydroxybutyrate polymer is selected from the group consisting of poly-(*R*)- 3-hydroxybutyrate and poly-4-hydroxybutyrate.

According to another preferred embodiment of the present invention the polyhydroxybutyrate polymer is provided as a powder, particle, flake, slurry, fiber, sheet, pellet or a combination thereof.

The amount of the provided least one solid polyhydroxybutyrate polymer can be adjusted accordingly to reach a sufficient final concentration of enantiopure (R)- 3-hydroxybutyrate acid or a salt thereof. According to another preferred embodiment of the present invention the concentration of the at least one polyhydroxybutyrate polymer in the aqueous solution of step b) is between 50 and 500 g/L, preferably between 50 and 400 g/L, preferably between 100 and 350 g/L, preferably between 150 and 300 g/L.

The first and the second enzyme of the present invention can be mixed at a defined weight ratio. This ratio may be one factor which influences the product inhibition of the enzymes. If more of the first enzyme is added, more product can be formed at higher amounts of (R)- 3-hydroxybutyrate. According to another preferred embodiment of the present invention the at least one polyhydroxybutyrate polymer and the at least one first polyhydroxybutyrate depolymerase and/or the at least one second polyhydroxybutyrate depolymerase are mixed at a weight ratio in the range of 10000:1 to 100:1, preferably 8000:1 to 200:1, preferably 6000:1 to 400:1, preferably 5000:1 to 500:1, preferably 2000:1 to 800:1.

The release velocity of the enantiopure (*R*)- 3-hydroxybutyrate acid or a salt thereof from PHB is a critical parameter in the production processes. The higher the release velocity, the higher the enzymatic activity and thus, the more product can be obtained in defined period of time. According to another preferred embodiment of the present invention the release velocity of enantiopure (*R*)- -hydroxybutyrate acid or a salt thereof from the at least one polyhydroxybutyrate polymer is between 10 to 100 pmol/min/mg, preferably between 20 to 80 pmol/min/mg, more preferably between 30 to 70 pmol/min/mg, even more preferably between 40 to 70 pmol/min/mg, most preferably between 50 and 60 pmol/min/mg.

The obtained product may also have an inhibitory effect on the activity on the polyhydroxybutyrate depolymerases of the present invention. Thus, attention should be drawn to the final concentration of enantiopure (*R*)- 3-hydroxybutyrate acid or a salt thereof in the aqueous solution. According to another preferred embodiment of the present invention the concentration of enantiopure (*R*)- 3-hydroxybutyrate acid or a salt thereof in the aqueous solution is between 50 and 500 g/L, preferably between 100 to 400 g/L, more preferably between 100 t0 300 g/L, even more preferably between 150 to 200 g/L.

According to another preferred embodiment of the present invention the enantiopure (*R*)- 3-hydroxybutyrate acid or a salt thereof is recovered from the aqueous solution by filtration, absorption, adsorption or combinations thereof.

According to another further preferred embodiment of the present invention the at least one first and/or the at least second polyhydroxybutyrate depolymerase is recovered from the aqueous solution by filtration, wherein the filtration is performed by using a polyhydroxybutyrate polymer as filtration aid. The polyhydroxybutyrate polymer as filtration aid can bind the remaining polyhydroxybutyrate depolymerase. Preferably,the polyhydroxybutyrate depolymerase as a filter aid is a powder. The filtration aid can be used in two different ways. They can be used either as a precoat of the filter before the aqueous reaction mixture is filtered, or they can be added to the aqueous solution before filtration.

The polyhydroxybutyrate polymer and the bound polyhydroxybutyrate depolymerase can be subsequently recycled into the hydrolysis reaction. Especially with the reaction performed in a membrane reactor it is of advantage to use a polyhydroxybutyrate depolymerase having a polymer binding activity as this facilitates keeping the enzyme bound to the polymer and thus the enzyme is better retained in the reaction thereby reducing loss of enzyme by transfer across the membrane.

### EXAMPLES

### Example 1: Identification and isolation of a polyhydroxybutyrate depolymerase and construction of an expression vector

To obtain an active depolymerase a gene library of a polyhydroxybutyrate-degrading bacterium (Acidovorax species BT293) isolated from soil samples was created. The genomic DNA of the mentioned bacterium from soil samples was isolated and partially restricted by Sau3A I. The obtained DNA-fragments were cloned into the screening vector [pZeroTM-2, ThermoFisher]. This vector was subsequently transformed to E. coli TOP10 by electroporation. The clones were plated out on selection agar plates containing suspended PHB powder (10 g/L) and screened for PHB degradation at 37°C (incubation time 12 to 28 h). If a recombinant clone was able to degrade PHB, a clear zone appeared where the substrate was degraded. The colony with the highest degrading activity was isolated and the insert was sequenced. Several open reading frames were detected and one with sequence characteristics of a putative polyhydroxybutyrate depolymerase was identified by BLAST searches. The DNA fragment encoding for this polyhydroxybutyrate depolymerase (designated as TP0001, SEQ-ID 1) was amplified by PCR and introduced into a tac-promoter based expression vector, [pMS470-C termHis] (based on Balzer et al., Nucleic Acids Research, 20(1992):1851-1858). By cloning the fragment into this vector, a His-tag on the C-terminus of the protein was added allowing efficient purification. The sequenced gene encoding polyhydroxybutyrate depolymerase has a subtype B catalytic domain and a SBD (substrate binding domain) type I. All reported catalytic amino acid sequences such as the oxyanion-hole as well as a catalytic aspartic and a catalytic histidine are present in this protein. The enzyme has not yet been described in literature but shares 99% amino acid identity with a protein of Acidovorax sp. KKS102 (NCBI Reference Sequence: WP_015014950.1). A protein Blast search performed with the protein sequence of TP0001 (SEQ-ID 1) revealed that at amino acid position 121 only with TP0001 a threonine is present (121T) among the 100 sequences of nearest sequence homology. All other sequences have a different amino acid at this position (66 have 121N, 13 have 121S 10 have 121A, 8 have 121G and one each have 121E, 121Q and 121D).

The PHB-DP identified has the following amino acid sequence (SEQ ID No. 1):

MAFNFIRAAAAGAAMALCGAGGVHAAVNLPALKIDKTQTTVSGLSSGGFMAVQLHVAYSATF
AKGAGVVAGGPFYCAEGSIVNATGRCMASPAGIPTSTLVSTTNTWASQGVIDPVANLQTSKV
YLFSGTLDSVVKTGVMDALRTYYNSFVPAANVVYKKDIAAEHAMVTDDYGNACSTKGAPYIS
DCNFDLAGAMLQHLYGTLNARNNATLPTGNYIEFNQSEFITNHGMATTGWAYVPQACQAGGT
ATCKLHVVLHGCKQNISDVQQQYVRNTGYNRWADTNNIVMLYPQTSTAATNSCWDWWGYDSA
NYSKKSGPQMAAIKAMVDRVSSGTGGTTPPDPVALPAPTGVSTSGATASSMAIGWAAVTGAA

SYNVYRNANKVNALPVTATSYTDTGLAASTTYSWTVRAADANGAEGATSAAASGTTLAASGG
GTATCTTASNYAHTLAGRAYAAGGYTYALGSNQNMGLWNVFVTNTLKQTSTNYYVIGTCP

According to the analysis of the amino acid sequence with the SignalP program (Petersen et al.,Nature Methods, 8:785-786, 2011)the protein contains a signal peptide which gets processed by proteolytic cleavage during the export from the cell into the external space. The resulting mature protein has thus the following amino acid sequence (SEQ ID No. 2).

AVNLPALKIDKTQTTVSGLSSGGFMAVQLHVAYSATFAKGAGVVAGGPFYCAEGSIVN
ATGRCMASPAGIPTSTLVSTTNTWASQGVIDPVANLQTSKVYLFSGTLDSVVKTGVMDALRT
YYNSFVPAANVVYKKDIAAEHAMVTDDYGNACSTKGAPYISDCNFDLAGAMLQHLYGTLNAR
NNATLPTGNYIEFNQSEFITNHGMATTGWAYVPQACQAGGTATCKLHVVLHGCKQNISDVQQ
QYVRNTGYNRWADTNNIVMLYPQTSTAATNSCWDWWGYDSANYSKKSGPQMAAIKAMVDRVS
SGTGGTTPPDPVALPAPTGVSTSGATASSMAIGWAAVTGAASYNVYRNANKVNALPVTATSY
TDTGLAASTTYSWTVRAADANGAEGATSAAASGTTLAASGGGTATCTTASNYAHTLAGRAYA
AGGYTYALGSNQNMGLWNVFVTNTLKQTSTNYYVIGTCP

Both, the unprocessed and processed forms are enzymatically active. The PHB-DP containing a His-Tag at the C-terminus to facilitate purification has the following amino acid sequence (SEQ ID No. 3, the signal sequence is underlined and put in parentheses as it will be not present in the processed protein, a short linker sequence between the coding sequence and the his-tag is marked by italicized letters):

MAFNFIRAAAAGAAMALCGAGGVHAAVNLPALKIDKTQTTVSGLSSGGFMAVQLHVAY
SATFAKGAGVVAGGPFYCAEGSIVNATGRCMASPAGIPTSTLVSTTNTWASQGVIDPVANLQ
TSKVYLFSGTLDSVVKTGVMDALRTYYNSFVPAANVVYKKDIAAEHAMVTDDYGNACSTKGA
PYISDCNFDLAGAMLQHLYGTLNARNNATLPTGNYIEFNQSEFITNHGMATTGWAYVPQACQ
AGGTATCKLHVVLHGCKQNISDVQQQYVRNTGYNRWADTNNIVMLYPQTSTAATNSCWDWWG
YDSANYSKKSGPQMAAIKAMVDRVSSGTGGTTPPDPVALPAPTGVSTSGATASSMAIGWAAV
TGAASYNVYRNANKVNALPVTATSYTDTGLAASTTYSWTVRAADANGAEGATSAAASGTTLA
ASGGGTATCTTASNYAHTLAGRAYAAGGYTYALGSNQNMGLWNVFVTNTLKQTSTNYYVIGT
CPKLAAALEHHHHHH*

### Example 2: Expression and purification

Expression was performed in E. coli XL1 by cultivation over 24h without induction by IPTG. The fermentation broth was centrifuged, the cell pellet washed and resuspended in 100 mM potassium phosphate buffer, pH 7.4 and cells were disrupted by ultrasonic treatment. The cell debris and insoluble components were removed by centrifugation (20,000 x g) and the supernatant sterile filtered (0.2 pm) prior protein purification. This supernatant contained intracellular and periplasmic proteins and thus contains both processed and unprocessed polyhydroxybutyrate depolymerase protein. Protein purification was performed according to the standard protocol for His-tagged proteins (GE Healthcare, Ni Sepharose FastFlow). The buffer containing imidazole was exchanged directly after purification against 100 mM potassium phosphate buffer, pH 7.4 by gel filtration.

In an alternative strategy the expression of the polyhydroxybutyrate depolymerase was completely directed to intracellular expression. Therefore, the signal sequence was removed (from amino acid 2 to 25) and remaining the his-tag resulting in a protein defined by SEQ ID No. 4.

MAVNLPALKIDKTQTTVSGLSSGGFMAVQLHVAYSATFAKGAGVVAGGPFYCAEGSIV
NATGRCMASPAGIPTSTLVSTTNTWASQGVIDPVANLQTSKVYLFSGTLDSVVKTGVMDALR
TYYNSFVPAANVVYKKDIAAEHAMVTDDYGNACSTKGAPYISDCNFDLAGAMLQHLYGTLNA
RNNATLPTGNYIEFNQSEFITNHGMATTGWAYVPQACQAGGTATCKLHVVLHGCKQNISDVQ
QQYVRNTGYNRWADTNNIVMLYPQTSTAATNSCWDWWGYDSANYSKKSGPQMAAIKAMVDRV
SSGTGGTTPPDPVALPAPTGVSTSGATASSMAIGWAAVTGAASYNVYRNANKVNALPVTATS
YTDTGLAASTTYSWTVRAADANGAEGATSAAASGTTLAASGGGTATCTTASNYAHTLAGRAY
AAGGYTYALGSNQNMGLWNVFVTNTLKQTSTNYYVIGTCPKLAAALEHHHHHH*

The respective DNA fragment was cloned into the vector pCM470-SS-DsbC, which is a derivative of pMS470, but containing a chloramphenicol resistance gene for selection and a dsbC gene from E.coli which encodes for a disulfide bond isomerase. The resulting expression plasmid was transformed into an E.coli Origami B strain (Novagen). Colonies grown on LB agar plates supplemented with 25 mg/L chloramphenicol and 10 mg/L tetracycline were used to inoculate 30 mL LB medium (300 mL flask) supplemented with 25 mg/L chloramphenicol. This pre-culture was incubated overnight at 37 °C on a shaker (150 rpm) and used for the inoculation of 300 mL LB medium supplemented with 25 mg/L chloramphenicol in a 1000 mL flask to an OD=0.1. The main culture was then incubated under the same conditions at 37°C to an OD=1.0, thereafter the temperature was lowered to 25°C, and the expression was induced by addition of 0.1 mM IPTG and cultivation was continued for 16 hours. Harvesting and purification was performed as described above.

For comparison, four additional proteins (Table 1) having high homology to TP0001 were also cloned and expressed with this alternative intracellular method. The respective DNA fragment encoding variant TP0003 was obtained as synthetic DNA based on codon optimized sequence for optimal expression in E.coli. The DNA fragments encoding other variants were generated by replacing the differing amino acids by changing the codons at the respective positions using the overlap extension PCR strategy and the gene sequence encoding fpr TP0001 as template.

**Table 1: Summary of expressed PHB-DP variants. TP0002 and TP0003 and TP0005 are designated as PHB-DPs, TP0004 was annotated as hypothetical protein.**

| **Protein Index** | **Source Organism** | **Genebank Swissprot** | **MW [kDa]** | **AA** | **Variations to TP0001** |
|---|---|---|---|---|---|
| TP0001 | Acidovorax sp. BT293 | | 51.8 | 500 | |
| TP0002 | Delftia acidovorans | BAA19791.1 | 51.0 | 494 | **T121N,** S265G, T269M |
| TP0003 | Acidovorax sp. TP4 | BAA35137.1 | 51.0 | 491 | A66V, I81V, A93T, S102N, S109G, **T121N,** A164S, A176G, T214S, Y217F, T227S, I264V, S265N, T283S, T295L, S331-, S332-, T334S, P339N, D341-, A344D, S352K, T353P, A366N, T369S, A371G, A372P, A380G, V383T, S392N, Y393F, A413G, T420V, A437G, L449M, A455V, A456Y, Y461F, N468S, V474I, T477N, N478K, T485P, |
| TP0004 | Acidovorax sp. KKS102 | WP_0150149 50.1 | 51.0 | 494 | **T121N,** S359N |
| TP0005 | Acidovorax sp. BE320 | MBP2141865 .1 | 51.1 | 494 | A20V, G22S, T61M, K64R, **T121N,** A399T |

### Example 3: Activity Assay

10 mg of PHB powder (Enmat Y3000, TianAn Biopolymer, Ningbo City, China) were placed in a 2 mL polypropylene tube and mixed with 1 mL enzyme solution in 200 mM potassium phosphate buffer, pH 7.4. The samples were incubated at 25°C in a shaking incubator (550 rpm). Samples (40 pL) were taken in time intervals, centrifuged at 10,000 x g, and the supernatants collected and kept frozen at -20°C until further use. For determination of activity, the frozen samples were thawed and the amount of released 3-hydroxybutyrate was determined by an enzymatic method employing a ready to use reagent kit (Megazyme, Product code K-HDBA, www.megazyme.com).

### Example 4: Induction of secretory production of polyhydroxybutyrate depolymerase during cultivation of Acidovorax species BT293

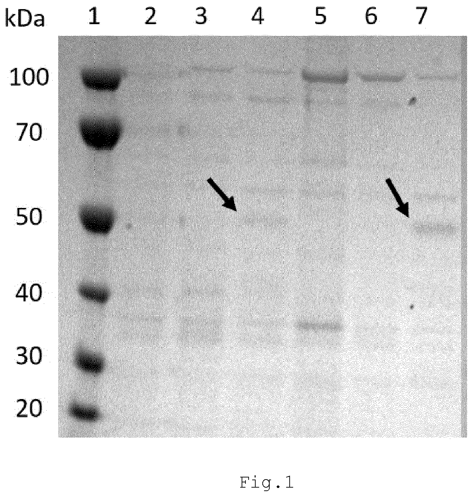

The PHB-degrading bacterium Acidovorax species BT293 was inoculated in 30 mL 1:50 diluted LB medium in 100 mL shake flasks. One part of the cultures was induced by addition of 0.1 g/L 3-hydroxybutyrate sodium salt, one part was induced by addition one 1 g/L PHB powder (Enmat Y3000, TianAn Biopolymer, Ningbo City, China), a third part was left without adding an inducing substance. The cultures were incubated on a laboratory shaker at 28°C or 37°C for 3 days and the cells removed by centrifugation. 13 µL of the supernatants were denatured under reducing conditions by heating with loading buffer and applied on SDS-polyacrylamide gels. The results summarized in Fig. 1 clearly demonstrate induction of a protein having the size of the polyhydroxybutyrate depolymerase in cultures where PHB was added as inducer.

### Example 5: Enzymatic hydrolysis of PHB particles with polyhydroxybutyrate depolymerase TP0001

10 g of PHB were suspended in 50 mL (200 g/L) water in a 100 mL reaction vessel of an autotitrator (Mettler Toledo T50). The reaction was started by addition of 10 mg polyhydroxybutyrate depolymerase (his tagged, purified, as specified in Examples 1 and 2) and a pH 8.5 was kept constant by autotitration with 1N NaOH. The reaction was run for 21.37 h at room temperature. The results are summarized in Table 2.

**Table 2: Enzymatic hydrolysis of 10 g PHB particles with purified recombinant polyhydroxybutyrate depolymerase TP0001. V_{NaOH}: volume of 1 N NaOH needed for pH correction; Vₜₒₜₐₗ: total liquid volume of reaction mixture; 3-HB-acid: 3-hydroxybutyric acid; 3-HB-Na: 3-hydroxybutyrate sodium salt.**

| **Time [h]** | **V_{NaOH} [mL]** | **3-HB-acid [g]** | **3-HB-Na [g]** | **Vₜₒₜₐₗ [mL total]** | **3-HB-acid [g/L]** | **3-HB-acid g /L/h** | **3-HB -Na [g/L]** | **3-HB-Na g /L/h** |
|---|---|---|---|---|---|---|---|---|
| 3 | 41.3 | 4.29 | 5.24 | 91.3 | 47.0 | 15.7 | 57.4 | 19.1 |
| 6 | 58.3 | 6.06 | 7.40 | 108.3 | 56.0 | 9.3 | 68.4 | 17.1 |
| 12 | 75.3 | 7.83 | 9.56 | 125.3 | 62.5 | 5.2 | 76.3 | 5.9 |
| 21.37 | 86.2 | 8.96 | 10.94 | 136.2 | 65.8 | 3.1 | 80.3 | 3.8 |

### Example 6: Enzymatic hydrolysis of PHB powder with polyhydroxybutyrate depolymerase TP0001 at high PHB load

20 g of PHB were suspended in 70 mL water (in a 100 mL reaction vessel of an autotitrator (Mettler Toledo T50). The reaction was started by addition of 10 mg PHB-DP (his tagged, purified, as specified in Examples 1 and 2) and a pH 8.5 was kept by autotitration with 30% (9.958 mol/L) NaOH. The reaction was run for 21.37 h at room temperature. The results are summarized in Table 3

**Table 3: Enzymatic hydrolysis of PHB particles with purified recombinant polyhydroxybutyrate depolymerase TP0001 at high PHB load. V_{NaOH}: volume of 30% NaOH needed for pH correction; Vₜₒₜₐₗ: total liquid volume of reaction mixture; 3-HB-acid: 3-hydroxybutyric acid; 3-HB-Na: 3-hydroxybutyrate sodium salt.**

| **Time [h]** | **V_{NaOH} [mL]** | **3-HB-(acid) [g]** | **3-HB-Na [g]** | **Vₜₒₜₐₗ [mL total]** | **3-HB (acid) [g/L]** | **3-HB (acid) g /L/h** | **3-HB -Na [g/L]** | **3-HB-Na g /L/h** |
|---|---|---|---|---|---|---|---|---|
| 3 | 5.3 | 5.48 | 6.70 | 75.3 | 72.8 | 24.3 | 88.9 | 29.6 |
| 4 | 6.0 | 6.21 | 7.59 | 76 | 81.7 | 20.4 | 99.8 | 25.0 |
| 13 | 9.8 | 10.19 | 12.44 | 79.84 | 127.6 | 9.8 | 155.8 | 12.0 |
| 21.7 | 12.2 | 12.66 | 15.46 | 82.23 | 154.0 | 7.1 | 188.1 | 8.7 |
| 24 | 12.6 | 13.06 | 15.95 | 82.61 | 158.1 | 6.6 | 193.0 | 8.0 |

### Example 7: Comparison of the enzymatic features of TP0001 and 4 variants

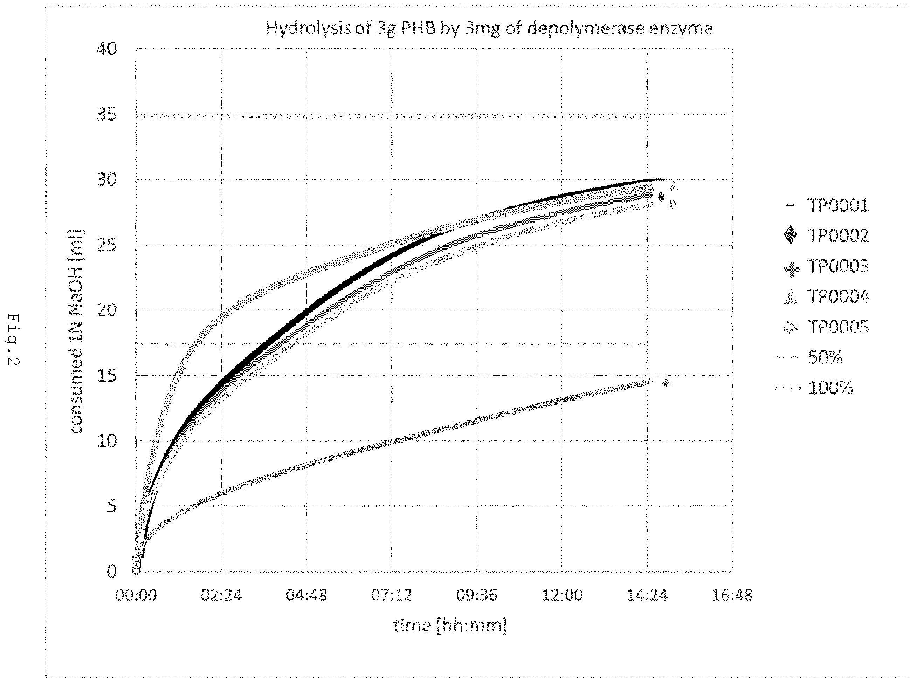

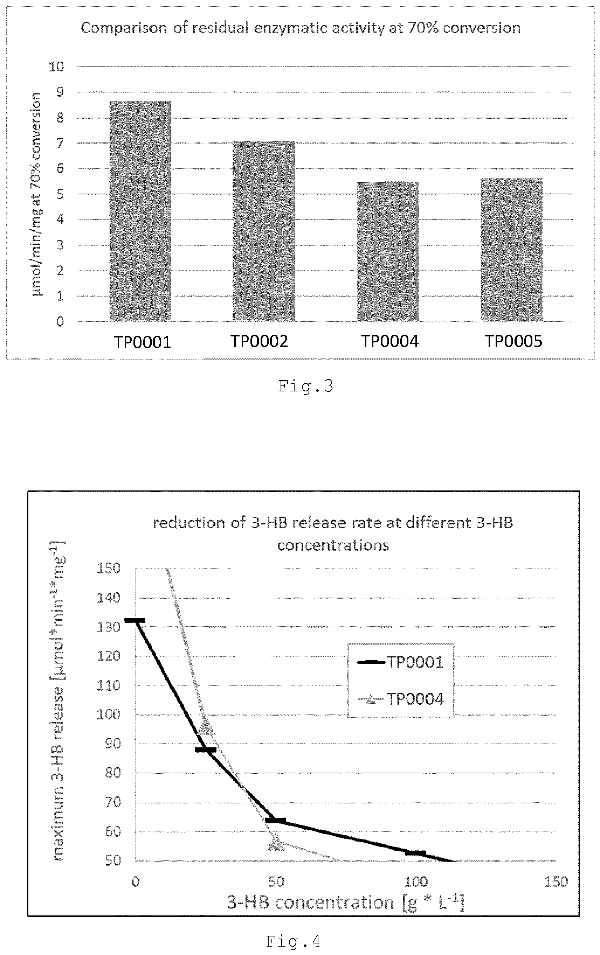

Reactions were set up by suspending 3 g PHB (same as with example 4) in 31 mL of 32 mM potassium phosphate buffer (pH 8.5). The reactions were started by addition of 3 mg purified PHB-DP and maintained at 30°C at constant pH by autotitration with 1 M NaOH. The results are summarized in Figure 2. As main outcome, TP0001 reaches highest degradation levels, TP0004 has high reactivity at low concentrations of the monomer 3-hydroxybutyrate, but shows significantly lower reactivity at higher 3-hydroxybutyrate concentrations. Fig. 3 shows the reactivity of the different PHB-DPs at 70% conversion of the PHB to 3-hydroxybutyrate.

### Example 8: Determination of 3-hydroxybutyrate monomer inhibition effects on TP0001 and TP0004

To separate different effects of the de-polymerization process such as polymer accession and product inhibition, the product inhibition by 3-hydroxybutyrate of the two best performing enzymes TP0001 and TP0004 was determined. For this purpose, 1 g PHB was suspended in 31 mL 32 mM potassium phosphate buffer containing different amounts of (R)-3-hydroxybutyrate monomers. The reaction was started by adding 1 mg enzyme and maintained at 30°C at constant pH by autotitration with 1 M NaOH. The results are summarized in Fig. 4. It can be seen that TP0001 is less inhibited at higher (R)-3-hydroxybutyrate concentrations, which are more relevant for efficient process operation.

### Example 9: Pretreatment of PHB with base prior to enzymatic hydrolysis

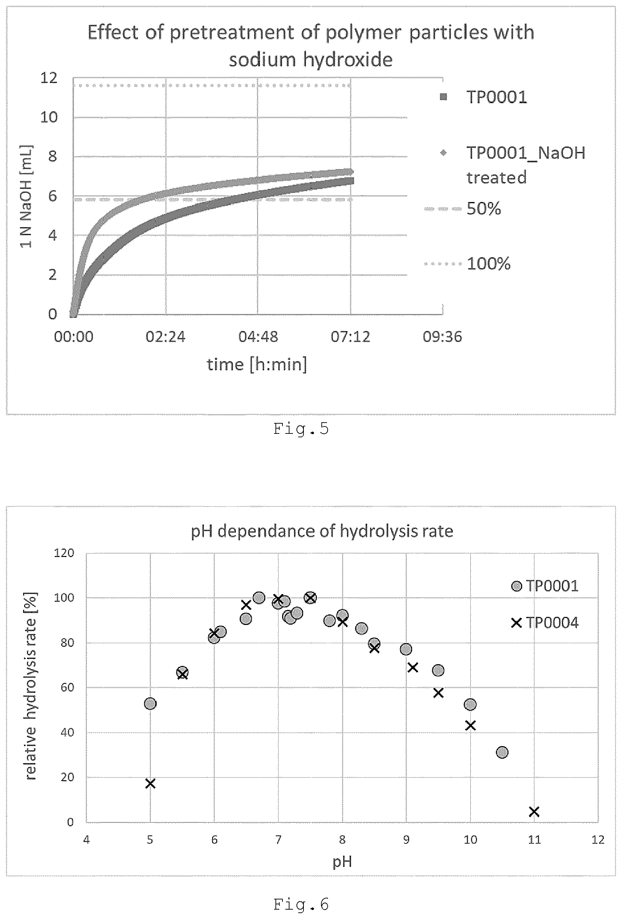

1 g of PHB powder was suspended in 30 ml H2O containing 0.5 molar equivalents of sodium hydroxide and incubated for 15 minutes at 100°C. The reaction mixture was cooled and set to pH=9.0 by addition of 5 mmol potassium di-hydrogen phosphate using a 1M stock solution. Thereafter 1 mg of TP0001 was added and the reaction run at constant pH=8.5 by autotitration with 1 N NaOH. The results summarized in Fig. 5 demonstrate that the hydrolysis rate with pre-treated PHB is significantly increased at low levels of released (R)-3-hydroxybutyrate monomer, but decreases at higher levels of released (R)-3-hydroxybutyrate monomer.

### Example 10: Determination of a suitable range of operating pH for TP0001

1 g PHB (Enmat Y3000) was suspended in 25 mL H2O plus 1 mL 1 M KH2PO4 and the pH was set by addition of 1N NaoH to the desired pH. Then 1 mg purified polyhydroxybutyrate depolymerase (TP0001 or TP0004) was added to start the hydrolysis reaction. The reaction was kept constant at the respective pH by autotitration with 1 N NaOH and the initial reaction velocity was determined at distinct pH values between 5.0 and 11.0 (Fig. 6). Both enzymes are well active within a rather broad pH range between pH 6 and pH 8.5. Reasonable activity is given in the acidic range down to pH 5.0, in the alkaline range up to pH 9.5.

## Claims

1. A polyhydroxybutyrate depolymerase for producing enantiopure (R)-3-hydroxybutyrate acid or a salt thereof comprising an amino acid sequence having at least 80% identity to SEQ ID No. 2 and a motif comprising amino acid sequence ANLQXSKVYL (SEQ ID No. 5), wherein X is threonine, serine, glycine, glutamic acid, glutamine or aspartic acid.

2. The depolymerase according to claim 1, wherein X is threonine.

3. The depolymerase according to claim 1 or 2, wherein the motif comprises amino acid sequence selected from the group consisting of VIDPVANLQXSKVYLFSGT (SEQ ID No. 6), GVIDPVANLQXSKVYLFSGTLDSWKTGVM (SEQ ID No. 7), QGVIDPVANLQXSKVYLFSGTLDSVVKTGVMDALRTYYNSF (SEQ ID No. 8), and QGVIDPVANLQXSKVYLFSGTLDSVVKTGVMDALRTYYNSFVPAANVVYKKDIA (SEQ ID No. 9) .

4. The depolymerase according to any one of claims 1 to 3, wherein the amino acid sequence has at least 85% identity, preferably at least 90% identity, more preferably at least 95% identity, more preferably at least 99% identity, to SEQ ID No. 2.

5. A nucleic acid molecule encoding the polyhydroxybutyrate depolymerase according to any of claims 1 to 4, wherein the nucleic acid molecule is operably linked to a promoter.

6. A vector comprising the nucleic acid molecule according to claim 5.

7. A host cell comprising the nucleic acid molecule of claim 5 and/or the vector of claim 6.

8. A method for producing enantiopure (*R*)-3-hydroxybutyrate acid or a salt thereof comprising the steps of:
a) providing at least one solid polyhydroxybutyrate polymer, and
b) incubating in an aqueous solution said at least one polymer
- with at least one first polyhydroxybutyrate depolymerase according to any one of claims 1 to 4 and/or a host cell according to claim 7 and/or
- with at least one second polyhydroxybutyrate depolymerase comprising an amino acid sequence having at least 80% identity to SEQ ID No. 2 and a motif comprising an amino acid sequence ANLQNSKVYL (SEQ ID No. 10).

9. The method according to claim 8, wherein before and/or during step b) the pH of the aqueous solution ranges from pH 5 to 10, preferably from pH 6 to 8, more preferably from pH 6.5 to 7.5.

10. The method according to claim 8 or 9, wherein the motif of the at least one second polyhydroxybutyrate depolymerase comprises amino acid sequence selected from the group consisting of VIDPVANLQNSKVYLFSGT (SEQ ID No. 11), GVIDPVANLQNSKVYLFSGTLDSVVKTGVM (SEQ ID No. 12), QGVIDPVANLQNSKVYLFSGTLDSVVKTGVMDALRTYYNSF (SEQ ID No. 13), and QGVIDPVANLQNSKVYLFSGTLDSVVKTGVMDALRTYYNSFVPAANVVYKKDIA (SEQ ID No. 14).

11. The method according to any of claims 8 to 10, wherein the at least one first and/or the at least one second polyhydroxybutyrate depolymerase comprises a further motif comprising amino acid sequence GATANSMAIG SEQ ID No. 15, or SGATANSMAIGW (SEQ ID No. 16).

12. The method according to any one of claims 8 to 11, wherein the at least one second polyhydroxybutyrate depolymerase comprises or consists of an amino acid sequence having at least 85% identity, preferably at least 90% identity, more preferably at least 95% identity, more preferably at least 99% identity, to SEQ ID No. 2.

13. The method according to any one of claims 8 to 12, wherein the at least one polyhydroxybutyrate polymer and the at least one first polyhydroxybutyrate depolymerase and/or the at least one second polyhydroxybutyrate depolymerase are mixed at a weight ratio in the range of 10000:1 to 100:1, preferably 8000:1 to 200:1, preferably 6000:1 to 400:1, preferably 5000:1 to 500:1, preferably 2000:1 to 800:1.

14. Use of a polyhydroxybutyrate depolymerase according to any of claims 1 to 6 or as defined in any one of claims 8 to 13 for producing enantiopure (*R*)-3-hydroxybutyrate acid or a salt thereof.

15. A food supplement or a kit comprising a first polyhydroxybutyrate depolymerase according to any one of claims 1 to 4 and/or a second polyhydroxybutyrate depolymerase as defined in any one of claims 8 to 12 and a polyhydroxybutyrate polymer.
